# EUROPEAN PATENT APPLICATION

(11) **EP 3 088 003 A1**
(43) Date of publication of application: **02.11.2016**
(21) Application number: 14874871.8
(22) Date of filing: 25.12.2014
(51) Int. Cl.: A61K 47/26, A61K 9/08, A61K 9/10, A61K 31/444, A61K 47/36, A61P 27/00

(54) **INJECTABLE AGENT AND DEPOT FORMATION METHOD**

(30) Priority: 25.12.2013 JP 2013266567
(71) Applicant: Santen Pharmaceutical Co., Ltd, Osaka-shi Osaka 533-8651 (JP)
(72) Inventor: OKI, Kenji, Ikoma-shi Nara 630-0101 (JP); YAMADA, Kazuhito, Ikoma-shi Nara 630-0101 (JP); MIYAZAKI, Tatsuya, Ikoma-shi Nara 630-0101 (JP); OKABE, Komei, Ikoma-shi Nara 630-0101 (JP); HABASHITA, Sayo, Ikoma-shi Nara 630-0101 (JP); ENOMOTO, Hiroshi, Ikoma-shi Nara 630-0101 (JP); YOSHII, Kenta, Ikoma-shi Nara 630-0101 (JP)
(74) Representative: Müller-Boré & Partner Patentanwälte PartG mbB
(86) International application number: PCT/JP2014/084289
(87) International publication number: WO 2015/099029

(57) **Abstract**

The purpose of the present invention is to provide a novel injectable agent having an excellent medicinal agent sustained release property. The injectable agent according to the present invention comprises a medicinal agent, trehalose and gellan gum. It is preferred that the contents of the medicinal agent, trehalose and gellan gum are 0.0001 to 50.00% (w/v), 0.1 to 50.0% (w/v) and 0.01 to 3.00% (w/v), respectively, relative to the total volume of the injectable agent. It is also preferred that the injectable agent can form a depot upon exposure to a phosphate buffer solution or a body fluid (tear fluid, vitreous fluid, etc.).

## Description

### TECHNICAL FIELD

The present invention relates to an injectable agent and a method of forming a depot.

### BACKGROUND ART

Diseases in the posterior segments of the eye such as the retina, choroid, optic nerve, vitreous body and sclera are often intractable. Therefore, there has been a demand for development of an effective medicinal agent treatment method thereof. Eye diseases are commonly treated by eye-drop administration of a medicinal agent, but the medicinal agent will not be easily translocated into the posterior segments of the eye such as the retina, choroid, optic nerve, vitreous body and sclera.

Accordingly, injectable agents for intravitreal injection, subconjunctival injection and the like have gathered attention as an administrative medicinal agent against diseases in the posterior segments of the eye. Administration of an injectable agent may, however, place a significant burden on a patient, and thus the concentration of a medicinal agent at the affected area is desirably maintained for a prolonged time in order to avoid frequent administrations. For example, Patent Document 1 discloses a formulation for intraocular administration which is formulated for sustained-release of a sulfonyl benzimidazole derivative as a medicinal agent.

Patent Document 1: Japanese Unexamined Patent Application (Translation of PCT Application), Publication No. 2009-536918

### DISCLOSURE OF THE INVENTION

### Problems to be Solved by the Invention

The present invention is made in view of the above circumstances, and an objective of the present invention is to provide a novel injectable agent having superior sustained-releasability of a medicinal agent.

### Means for Solving the Problems

(1) An injectable agent comprising a medicinal agent, trehalose and gellan gum.
(2) The injectable agent according to (1), wherein the content of the medicinal agent is 0.0001 to 50.00% (w/v), and the content of trehalose is 0.1 to 50.0% (w/v), and the content of gellan gum is 0.01 to 3.00%(w/v) relative to the total volume of the injectable agent.
(3) The injectable agent according to (1) or (2), wherein the injectable agent forms a depot upon exposure to a phosphate buffer solution, a body fluid or a pseudo-body fluid.
(4) The injectable agent according to any one of (1) to (3), wherein the injectable agent is intended for intravitreal administration.
(5) The injectable agent according to any one of (1) to (4), wherein the injectable agent is used to deliver the medicinal agent to retina and choroid.
(6) A method of forming a depot, the method comprising:
   exposing a liquid composition comprising a medicinal agent,
   trehalose and gellan gum to a body fluid, a pseudo-body fluid or a phosphate buffer solution.

### Effects of the Invention

According to the present invention, an injectable agent having superior sustained-releasability of a medicinal agent can be provided by virtue of including a combination of trehalose and gellan gum in addition to the medicinal agent.

### BRIEF DESCRIPTION OF THE DRAWINGS

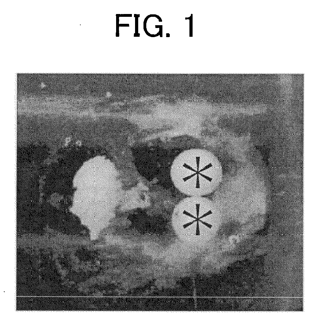

Fig. 1 shows a depot formed after intravitreal administration of the injectable agent according to one Example of the present invention.

### PREFERRED MODE FOR CARRYING OUT THE INVENTION

Below, embodiments of the present invention will be described, but the present invention shall not be limited to these.

The injectable agent according to the present invention contains a medicinal agent, trehalose and gellan gum. An injectable agent having superior sustained-releasability of a medicinal agent can be provided by virtue of including a combination of trehalose and gellan gum in addition to the medicinal agent. The present inventors find that trehalose can not only increase the wettability of a medicinal agent (in particular, a water-repellent medicinal agent) and can be excellent in isotonizing an injectable agent but also can improve medicinal agent dispersibility. The present inventors also found that gellan gum can form a good depot at a site where an injectable agent is administered, and the resulting depot can serve to effect sustained-release of a medicinal agent. Presumably, the integrity of a depot is also enhanced due to the coexistence of trehalose to show superior sustained-release of a medicinal agent.

Trehalose used in the present invention may be comprised of one or more of α,α-trehalose, α,β-trehalose and β,β-trehalose, and it preferably comprises α,α-trehalose. The content of trehalose relative to the total volume of an injectable agent is preferably 0.1% (w/v) or more, more preferably 1.0% (w/v) or more, 3.0% (w/v) or more, 4.0% (w/v) or more, 4.5% (w/v) or more, 5.0% (w/v) or more in view of sufficiently obtaining the above effect. The content of trehalose as described above is preferably 50.0% (w/v) or less, more preferably 20.0% (w/v) or less, or 10.0% (w/v) or less in view of adjusting the osmotic pressure and the like of the injectable agent.

Further, the content of trehalose is also preferably optimized depending on the medicinal agent or the content of gellan gum in view of the above effect.

Note that the injectable agent according to the present invention may further contain a saccharide other than trehalose. Examples of such a saccharide can include saccharide compounds selected form the group that consists of monosaccharides to hexasaccharides, sugar alcohols thereof, hyaluronic acid, maltodextrin and polyvinylpyrrolidones.

The content of gellan gum relative to the total volume of an injectable agent is preferably 0.01% (w/v) or more, 0.03% (w/v) or more, or 0.05% (w/v) or more, and is more preferably 0.10% (w/v) or more, 0.20% (w/v) or more, 0.30% (w/v) or more, 0.40% (w/v) or more in view of sufficiently accomplishing the above effect. The content of gellan gum as described above is preferably 3.00% (w/v) or less, more preferably 2.0% (w/v) or less, 1.0% (w/v) or less in view of adjusting the viscosity and the like of the injectable agent.

Further, the content of gellan gum is also preferably optimized depending on the medicinal agent or the content of trehalose in view of the above effect. For example, in a case where the content of trehalose is 20% (w/v) or more, the content of gellan gum may be 0.01% (w/v) or more and 0.20% (w/v) or less. The above combination is particularly suitable for a case where betamethasone is included as a medicinal agent. In a case where the content of trehalose is less than 20% (w/v), the content of gellan gum may be 0.10% (w/v) or more and 3.00% (w/v) or less.

The gellan gum used in the present invention may be comprised of one or more of ion-sensitive deacylated gellan gum, or heat-sensitive native gellan gum. Deacylated gellan gum is more preferred in that temperature control of an injectable agent (temperature is increased for solation before administration) is not required.

A medicinal agent may be appropriately selected depending on a medicinal agent efficacy required for an injectable agent. Specifically, it may be dissolved in water, or may be poorly dissolved in water. Preferred medicinal agents can include, for example, tyrosine kinase inhibitors such as Tafetinib, SIM-817378, ACTB-1003, Chiauranib, CT-53608, Cinnamon, chim 4G8-SDIE, CEP-5214, IMC-1C11, CEP-7055, 3-[5-[2-[N-(2-Methoxyethyl)-N-methylamino]ethoxy]-1H-indol-2-yl]quinolin-2(1H)-one, hF4-3C5, ZK-CDK, IMC-EB10, LS-104, CYC-116, OSI-930, PF-337210, JNJ-26483327, SSR-106462, R-1530, PRS-050, TG-02, SC-71710, SB-1578, AMG-191, AMG-820, Sulfatinib, Lucitanib hydrochloride, JNJ-28312141, Ilorasertib, PLX-5622, ARRY-382, TAS-115, Tanibirumab, Henatinib maleate, LY-2457546, PLX-7486, FPA-008, NVP-AEE-788, cgi-1842, RAF-265, MK-2461, SG-00529, Rebastinib tosylate, Golvatinib tartrate, Roniciclib, BVT-II, X-82, XV-615, KD-020, Lestaurtinib, Delphinidin, Semaxanib, Vatalanib succinate, OSI-632, Telatinib, Alacizumab pegol, ATN-224, Tivozanib, XL-999, Icrucumab, Foretinib, Crenolanib besylate, R-406, Brivanib, Pegdinetanib, TG-100572, Olaratumab, Fostamatinib disodium, BMS-690514, AT-9283, MGCD-265, Quizartinib, ENMD-981693, Famitinib malate, Anlotinib, Tovetumab, PLX-3397, Fruquintinib, (-)-Epigallocatechin gallate, Midostaurin, NSC-706456, Orantinib, Cediranib, Dovitinib lactate, XL-647, Motesanib diphosphate, Linifanib, Brivanib alaninate, Cediranib maleate, Apatinib, Fedratinib, Pacritinib, Ramucirumab, Intedanib, Masitinib mesylate, Elemene, Dihydroartemisinin, WS-1442, Itraconazole, Leflunomide, Dihydroartemisinin, Imatinib mesylate, Sorafenib, Sunitinib malate, Dasatinib, Pazopanib hydrochloride, Vandetanib, Axitinib, Regorafenib, Cabozantinib S-malate, Ponatinib and 2-[[[2-[(hydroxyacetyl)amino]-4-pyridinyl]methyl]thio]-N-[4-(trifluoromethoxy)phenyl]-3-pyridine carboxamide; steroids such as hydrocortisone, triamcinolone, fluocinolone, dexamethasone and betamethasone; prostaglandins such as isopropyl unoprostone; immunosuppressive agents such as ciclosporin and sirolimus (rapamycin); antiallergic agents such as azelastine; nonsteroidal anti-inflammatory agents such as indomethacin, bromfenac and diclofenac; angiogenic inhibitors such as pazopanib, SU5416, valatinib, ranibizumab and bevacizumab; circulation activators such as nicardipine and nitrendipine; anti-oxidant agents such as vitamin E; carbonic anhydrase inhibitors such as acetazolamide and brinzolamide; intraocular pressure-lowering medicinal agents such as timolol and prostaglandin derivatives; visual cycle modulators such as various vitamin A derivatives; nutritional factors and nerve growth factors (NGF) such as ciliary neurotrophic factor (CNTF) and brain-derived neurotrophic factor (BDNF); various growth factors like hepatocyte growth factor (HGF); aptamers like pegaptanib; nucleic acid medicines like various antisense nucleic acids and siRNA; antibody/peptide formulations such as Lucentis and IgG; VEGF inhibitors described in Japanese Patent Application Laid-Open Nos. 2006-96739, 2011-37844, 2005-232149, 2006-273851, 2006-306861, 2008-266294 and so on; compounds having glucocorticoid receptor-binding activity described in Japanese Patent Application Laid-Open Nos. 2007-230993, 2008-074829, 2008-143889, 2008-143890, 2008-143891, 2009-007344, 2009-084274 and so on; selective glucocorticoid receptor agonists such as RU24858; anticancer medicinal agents such as fluorouracil; Janus kinase inhibitors such as tofacitinib; protein kinase inhibitors such as ruboxistaurin mesylate; and the like.

The content of the medicinal agent relative to the total volume of an injectable agent is preferably 0.0001% (w/v) or more, more preferably 0.05% (w/v) or more, 0.10% (w/v) or more, 0.30% (w/v) or more, 0.50% (w/v) or more, 1.0% (w/v) or more. Further, superior therapeutic/preventive effects can be obtained even with a relatively small amount of a medicinal agent because the injectable agent according to the present invention shows superior sustained-releasability of a medicinal agent. Therefore, the content of a medicinal agent as described above is preferably 50.00% (w/v) or less, more preferably 40.00% (w/v) or less, 30.00% (w/v) or less, or 25.00% (w/v) or less.

The injectable agent according to the present invention may also comprise a dispersion medium such as water, an osmotic pressure-adjusting agent, a buffer in addition to the aforementioned components.

There is no particular limitation for the injectable agent according to the present invention as long as it can form a depot to demonstrate sustained-releasability. According to the present invention, a depot formation may be initiated upon exposure to a phosphate buffer solution, a body fluid (the tear fluid, the vitreous fluid and the like), and appropriate formulations and modes of administration may be used depending on various conditions. For example, an injectable agent which forms a depot upon exposure to a body fluid (the tear fluid, the vitreous fluid and the like) may be administered by injection into the body or the vitreous body. It may also be administered into various test liquids such as a pseudo-body fluid and a phosphate buffer solution. This will allow a depot to be formed at each administration site where sustained-release of a medicinal agent will take place.

The injectable agent according to the present invention will form a depot in the vicinity of an administration site when administered, for example, into the vitreous body. Therefore, a medicinal agent can be delivered into an affected area inside the eye (for example, into retina and choroid) effectively and continuously.

In this case, the injectable agent according to the present invention can suitably be used for treating/preventing posterior ocular diseases. Posterior ocular diseases can include inflammations with various causes, viral and bacterial infections, vascularization in retina and choroid, diseases resulted from vascular hyperpermeability, neoplastic diseases of retina and choroid, genetic diseases of retina and choroid and optic nerve disorders resulted from glaucoma. More specifically, posterior ocular diseases can include endophthalmitis, uveitis, cytomegalovirus retinitis, age-related macular degeneration, diabetic retinopathy, proliferative vitreoretinopathy, retinal vein occlusion, retinal artery occlusion, retinal detachment, central serous chorioretinopathy, retinoblastoma, retinitis pigmentosa, macular dystrophy, choroideremia, daltonism, congenital retinoschisis, visual field constriction accompanied with glaucoma, visual field defect and the like. However, the injectable agent according to the present invention may be used to treat/prevent various diseases depending on medicinal agents or modes of administration.

The injectable agent according to the present invention can be manufactured by appropriate methods. For example, gellan gum is first added to a solvent to prepare a base agent. Any solvent may be used as long as it is physiologically acceptable, but distilled water for injection is preferably used. A medicinal agent is added to the above base agent, and uniformly dispersed, suspended or dissolved to prepare an injectable agent. Suspension of a medicinal agent into a base agent may be performed by thoroughly pulverizing a solid medicinal agent, and then performing uniform dispersion into the base agent, or may be performed by emulsifying a liquid medicinal agent. Emulsification methods can include the surface-chemical emulsification method, the mechanical emulsification method, the membrane emulsification method and the like.

The present invention also encompasses a method of forming a depot, the method comprising: exposing a liquid. composition containing a medicinal agent, trehalose and gellan gum to a body fluid, a pseudo-body fluid or a phosphate buffer solution.

### EXAMPLES

### [1] Preparation of Formulation Examples 1 to 3 and Comparative Examples 1 to 2

### Formulation Example 1

To a volumetric flask charged with 0.025 g of 2-[[[2-[(hydroxyacetyl)amino]-4-pyridinyl]methyl]thio]-N-[4-(trifluoromethoxy)phenyl]-3-pyridine carboxamide (hereinafter referred to as "the compound A"), 0.5 mL of 0.4% aqueous polyoxyethylene sorbitan monolaurate (Tween 20) was added, and dispersed with a stirrer and by ultrasound. Next, 0.8 mL of 50% sterilized aqueous trehalose was added and stirred with a stirrer, and then 2 mL of 1% aqueous deacylated gellan gum was added and again stirred with a stirrer, to which water for injection was further added to a volume of 5 mL for preparing an injectable agent.

### Formulation Example 2

To a volumetric flask charged with 0.125 g of the compound A, 0.5 mL of 0.4% aqueous polyoxyethylene sorbitan monolaurate (Tween 20) was added, and dispersed with a stirrer and by ultrasound. Next, 0.8 mL of 50% aqueous trehalose was added and stirred with a stirrer, and then 2 mL of 1% aqueous deacylated gellan gum was added and again stirred with a stirrer, to which water for injection was further added to a volume of 5 mL for preparing an injectable agent.

### Formulation Example 3

To a volumetric flask charged with 0.250 g of the compound, 0.5 mL of 0.4% aqueous polyoxyethylene sorbitan monolaurate (Tween 20) was added, and dispersed with a stirrer and by ultrasound. Next, 0.8 mL of 50% sterilized aqueous trehalose was added and stirred with a stirrer, and then 2 mL of 1% aqueous deacylated gellan gum was added and again stirred with a stirrer, to which water for injection was further added to a volume of 5 mL for preparing an injectable agent.

### Comparative Example 1

To a volumetric flask charged with 0.125 g of the compound A, 0.5 mL of 0.4% aqueous polyoxyethylene sorbitan monolaurate (Tween 20) was added, and dispersed with a stirrer and by ultrasound. Next, 0.8 mL of 50% aqueous trehalose was added and stirred with a stirrer, and water for injection was then added to a volume of 5 mL for preparing an injectable agent.

### Comparative Example 2

To a volumetric flask charged with 0.125 g of the compound A, 0.5 mL of 0.4% aqueous polyoxyethylene sorbitan monolaurate (Tween 20) was added, and dispersed with a stirrer and by ultrasound. Next, 0.8 mL of 1% aqueous deacylated gellan gum was added and again stirred with a stirrer, to which water for injection was further added to a volume of 5 mL for preparing an injectable agent. However, the resulting injectable agent was a heterogeneous suspension containing aggregates, and thus unusable for the following elution tests.

### [2] Elution test for each injectable agents obtained from Formulation Examples 1 to 3 and Comparative Examples 1 to 2

To each glass test tube charged with 50 mL of 10 mM neutral phosphate buffer solution (Dulbecco PBS) containing 1% polyethylene glycol monostearate (MYS40), 0.02 mL of each injectable agent obtained from Formulation Examples 1 to 3 and Comparative Examples 1 was added, and gently shaken at 37°C. Elutes each in an amount of 1 mL were collected 1 day and 3 days after the start of the elution tests, and the concentration of the compound A in each elute was computed from the absorbance at 270 nm with a microplate reader. Note that a calibration curve was created with 0.000313, 0.000625, 0.00125, 0.0025, 0.005 and 0.01 mg/mL solutions of the compound A in 10 mM neutral phosphate buffer solution (Dulbecco PBS) containing 1% MYS40. The test results are shown in Table 1.

**[Table 1]**

| | Concentration of Compound A | Concentration of trehalose | Concentration of gellan gum | Cumulative release rate(%) | |
|---|---|---|---|---|---|
| | | | | After 1 day | After 3 days |
| Formulation Example 1 | 0.5% | 8% | 0.4% | 56.4 | 82.3 |
| Formulation Example 2 | 2.5% | 8% | 0.4% | 14.9 | 29.6 |
| Formulation Example 3 | 5% | 8% | 0.4% | 21.6 | 35.4 |
| Comparative Example 1 | 2.5% | 8% | - | 75.0 | >100 |
| Comparative Example 2 | 2.5% | - | 0.4% | - | - |

As understood from Table 1, Formulation Examples 1 to 3 all showed a good sustained-release effect (medicinal agent release rate) for the medicinal agent (the compound A). In contrast, Comparative Example 1, which contains no gellan gum, does not show a good sustained-releasability of the medicinal agent. Comparative Example 2, which contains no trehalose, cannot be used as an injectable agent because it contains aggregates.

### [3] Preparation of Formulation Examples 4 to 8

### Formulation Example 4

To a volumetric flask charged with 50 mg of the compound A, 0.2 mL of 50% aqueous trehalose was added, and stirred and dispersed with a stirrer and by sonication. Then 0.8 mL of 1% aqueous deacylated gellan gum was added, and again stirred and dispersed to prepare an injectable agent.

### Formulation Example 5

To a volumetric flask charged with 50 mg of the compound A, 0.2 mL of 25% aqueous trehalose was added, and stirred and dispersed with a stirrer and by sonication. Then 0.8 mL of 1% aqueous deacylated gellan gum was added, and again stirred and dispersed to prepare an injectable agent.

### Formulation Example 6

To a volumetric flask charged with 50 mg of the compound A, 0.1 mL of 50% aqueous trehalose was added, and stirred and dispersed with a stirrer and by sonication. Then 0.9 mL of 1% aqueous deacylated gellan gum was added, and again stirred and dispersed to prepare an injectable agent.

### Formulation Example 7

To a volumetric flask charged with 50 mg of the compound A, 0.1 mL of 50% aqueous trehalose was added, and stirred and dispersed with a stirrer and by sonication. Then 0.6 mL of 1% aqueous deacylated gellan gum and 0.3 mL of water for injection were added, and again stirred and dispersed to prepare an injectable agent.

### Formulation Example 8

To a volumetric flask charged with 50 mg of the compound A, 0.1 mL of 50% aqueous trehalose was added, and stirred and dispersed with a stirrer and by sonication. Then 0.4 mL of 1% aqueous deacylated gellan gum and 0.5 mL of water for injection were added, and again stirred and dispersed to prepare an injectable agent.

### [4] Elution test for each injectable agent obtained from Formulation Examples 4 to 8

To each glass test tube charged with 50 mL of 10mM neutral phosphate buffer solution (Dulbecco PBS) containing 1% polyethylene glycol monostearate (MYS40), 0.02 mL of each injectable agent obtained from Formulation Examples 4 to 8 was added, and gently shaken at 37°C. Elutes each in an amount of 0.18 mL were collected 1 day, 3 days and 7 days after the start of the elution tests, and the concentration of the compound A in each elute was computed from the absorbance at 270 nm with a microplate reader. Note that a calibration curve was created with 0.0008, 0.004 and 0.02 mg/mL solutions of the compound A in 10 mM neutral phosphate buffer solution (Dulbecco PBS) containing 1% MYS40. The test results are shown in Table 2.

**[Table 2]**

| | Concentration of Compound A | Concentration of trehalose | Concentration of gellan gum | Cumulative release rate(%) | | |
|---|---|---|---|---|---|---|
| | | | | After 1 day | After 3 days | After 7 days |
| Formulation Example 4 | 5% | 10% | 0.8% | 27.0 | 52.4 | 67.5 |
| Formulation Example 5 | 5% | 5% | 0.8% | 24.2 | 40.7 | 59.4 |
| Formulation Example 6 | 5% | 5% | 0.9% | 17.5 | 37.6 | 56.2 |
| Formulation Example 7 | 5% | 5% | 0.6% | 26.0 | 44.5 | 60.0 |
| Formulation Example 8 | 5% | 5% | 0.4% | 45.6 | 55.4 | 71.7 |

As understood from Table 2, Formulation Examples 4 to 8 all showed a good sustained-release effect (medicinal agent release rate) for the medicinal agent (the compound A).

### [5] Preparation of Formulation Examples 9 and 10

### Formulation Example 9

To a volumetric flask charged with 30 mg of sirolimus, 0.1 mL of 50% aqueous trehalose was added, and dispersed with a stirrer and by sonication. Then 0.9 mL of 1% aqueous deacylated gellan gum was added, and again stirred and dispersed to obtain an injectable agent.

### Formulation Example 10

To a volumetric flask charged with 30 mg of sirolimus, 0.6 mL of 50% aqueous trehalose was added, and dispersed with a stirrer and by sonication. Then 0.4 mL of 1% aqueous deacylated gellan gum was added, and again stirred and dispersed to obtain an injectable agent.

### [6] Elution test for each injectable agents obtained from Formulation Examples 9 and 10

To each glass test tube charged with 12 mL of a neutral phosphate buffer solution containing 0.1% sodium dodecyl sulfate (SDS), 0.015 mL of each injectable agent obtained from Formulation Examples 9 and 10 was added, and gently shaken at 37°C. Elutes each in an amount of 10 mL were collected 1 day after the start of the elution tests, and the concentration of sirolimus in each elute was computed using UPLC. The test results are shown in Table 3.

**[Table 3]**

| | Concentration of sirolimus | Concentration of trehalose | Concentration of gellan gum | Cumulative release rate(%) |
|---|---|---|---|---|
| | | | | After 1 day |
| Formulation Example 9 | 3% | 5% | 0.9% | 46.4 |
| Formulation Example 10 | 3% | 30% | 0.4% | 51.1 |

As understood from Table 3, Formulation Examples 9 and 10 showed a good sustained-release effect (medicinal agent release rate) for the medicinal agent (sirolimus).

### [7] Preparation of Formulation Examples 11 to 13

### Formulation Example 11

To a sterilized volumetric flask charged with 0.3 g of the compound A, 3.75 mL of 20% sterilized aqueous trehalose was added, and stirred with a stirrer and by sonication. Then 9 mL of 1% sterilized aqueous deacylated gellan gum was added, and again stirred with a stirrer. Water for injection was further added to a volume of 15 mL for preparing an injectable agent.

### Formulation Example 12

To a sterilized volumetric flask charged with 0.25 g of the compound A, 0.5 mL of 0.4% sterilized aqueous polyoxyethylene sorbitan monolaurate (Tween 20) was added, and dispersed with a stirrer and by ultrasound. Further, 0.8 mL of 50% sterilized aqueous trehalose was added and stirred with a stirrer, and then 2 mL of 1% sterilized aqueous deacylated gellan gum was added, and again stirred with a stirrer, to which water for injection was further added to a volume of 5 mL for preparing an injectable agent.

### Formulation Example 13

To a sterilized volumetric flask charged with 1 g of sirolimus, 2 mL of 20% sterilized aqueous trehalose was added, and stirred with a stirrer and by sonication. Then 3 mL of 1% sterilized aqueous deacylated gellan gum was added, and again stirred with a stirrer, to which water for injection was further added to a volume of 5 mL for preparing an injectable agent.

### [8] Assessments for medicinal agent release profiles in vivo

Japanese white rabbits (N = 4) were intravitreally administered with 50 µL of each injectable agent obtained from Formulation Examples 11 to 13 using a 26-gauge needle. The vitreous body, the retina and the choroid were collected from the rabbits over time after administration (after four weeks, 12 weeks, 24 weeks), and the concentration of the medicinal agent was measured using LC-MS/MS. Results from these medicinal agent release-profile assessments are shown in Tables 4 to 6.

**[Table 4]**

| Formulation Example 11(Compound A: 2%) | | | |
|---|---|---|---|
| | Percentage remained in vitreous body(%) | Concentration in retina and choroid(µg/g) | Concentration in plasma(ng/mL) |
| After 4 weeks | 93.7 | 0.27 | 0.02 |
| After 12 weeks | 70.5 | 0.15 | Less than determination limit |
| After 24 weeks | 66.4 | 0.19 | Less than determination limit |

**[Table 5]**

| Formulation Example 12(Compound A: 5%) | | | |
|---|---|---|---|
| | Percentage remained in vitreous body(%) | Concentration in retina and choroid(µg/g) | Concentration in plasma(ng/mL) |
| After 4 weeks | 77.4 | 0.42 | 0.03 |
| After 12 weeks | 79.5 | 0.21 | 0.02 |

**[Table 6]**

| Formulation Example 13(Sirolimus: 20%) | | | |
|---|---|---|---|
| | Percentage remained in vitreous body(%) | Concentration in retina and choroid(µg/g) | Concentration in plasma(ng/mL) |
| After 4 weeks | 86.6 | 0.59 | 0.32 |
| After 12 weeks | 68.3 | 0.49 | Concentration in plasma |

In the case of the present injectable agents (Formulation Examples 11 to 13), the concentrations of the medicinal agent in retina and choroid are maintained at a certain level or more over 4 weeks after the intravitreal injection, and 77% or more of the medicinal agent remains in the vitreous body 4 weeks after the administration, and 68% or more of the medicinal agent remains in the vitreous body even 12 weeks after the administration. Therefore, the sustained-release effect of the medicinal agent is likely to last for a prolonged time. Moreover, the medicinal agent contained in Formulation Examples 11, 12 is different from that contained in Formulation Examples 13. This suggests that the present injectable agents can share a sustained-release effect even in a case where different medicinal agents are contained therein.

### [8] Depot forming potential of the injectable agent obtained from Formulation Examples 11

The injectable agent from Formulation Examples 11 was injected into the vitreous body of a rabbit. After 4 weeks, the eyeball was excised and frozen with dry ice. The anterior segment of the eye was then removed from the pars plana with a razor blade. Further, the vitreous body was separated from retina and choroid in a frozen state with forceps and the like, and a depot in the vitreous body was photographed after the vitreous body was thawed. The results are shown in Fig. 1. Note that asterisks in Fig. 1 represent beads (3 mm in diameter) used for crushing the vitreous body gel.

As shown in Fig. 1, a depot is formed inside the vitreous body (the whitish object visible around the center slightly in the left side), demonstrating that the injectable agent according to the present invention can form a depot upon exposure to the vitreous fluid.

### [8] Preparation of Formulation Examples 14 to 19 Formulation Example 14

To a volumetric flask charged with 50 mg of betamethasone, 1 mL of 50% aqueous trehalose was added, and dispersed with a stirrer and by ultrasound. Then, 3 mL of 1% aqueous deacylated gellan gum was added, and again stirred with a stirrer, to which water for injection was further added to a volume of 5 mL for preparing an injectable agent.

### Formulation Examples 15 to 19

Injectable agents of Formulation Examples 15 to 19 were each prepared using similar operations as in Formulation Example 14 except that 50 mg of diclofenac, 50 mg of indomethacin, 50 mg of brinzolamide, 50 mg of azelastine, 50 mg of IgG (from human serum) were used instead of 50 mg of betamethasone.

Formulation compositions of injectable agents from Formulation Examples 14 to 19 are each shown in Table 7.

**[Table 7]**

| | Medicinal agent (Concentration) | Concentration of trehalose | Concentration of gellan gum |
|---|---|---|---|
| Formulation Example 14 | Betamethasone (1%) | 10% | 0.6% |
| Formulation Example 15 | Diclofenac (1%) | 10% | 0.6% |
| Formulation Example 16 | Indomethacin (1%) | 10% | 0.6% |
| Formulation Example 17 | Brinzolamide (1%) | 10% | 0.6% |
| Formulation Example 18 | Azelastine (1%) | 10% | 0.6% |
| Formulation Example 19 | IgG (1%) | 10% | 0.6% |

### [9] Tests for depot formation

To each glass test tube charged with 15 mL of 10 mM neutral phosphate buffer solution (Dulbecco PBS) containing 1% polyethylene glycol monostearate (MYS40), 0.020 mL of each injectable agent from Formulation Examples 14 to 19 was added at 37°C. Formation of white depots was visually observed in all test tubes containing the injectable agents from Formulation Examples 14 to 19.

### [10] Preparation of Formulation Examples 20 to 23 Formulation Example 20

To a sample vial charged with 50 mg of betamethasone, 0.05 mL of 50% aqueous trehalose was added, and dispersed with a stirrer and by ultrasound. Then, 4 mL of 1% aqueous deacylated gellan gum was added, and again stirred with a stirrer to uniformity, and then transferred into a volumetric flask, to which water for injection was added to a volume of 5 mL for preparing an injectable formulation. Note that temperature was appropriately increased to 50°C when solidification occurred during preparation.

### Formulation Example 21

An agate mortar was used to disperse 1.5 g of betamethasone into 2 mL of 50% aqueous trehalose. Then 0.15 mL of 1% aqueous deacylated gellan gum was added, and agitated with a vortex or pencil-type mixer to uniformity, and then transferred into a volumetric flask. Further, 2 mL of 50% aqueous trehalose was added while performing rinse with the same solution, and diluted with water for injection to a volume of 5 mL to obtain an injectable agent.

### Formulation Example 22

An agate mortar was used to disperse 3 g of betamethasone into 5 mL of 50% aqueous trehalose. Then 1 mL of 1% aqueous deacylated gellan gum was added, and agitated with a vortex or pencil-type mixer to uniformity, and then transferred into a volumetric flask, and diluted with water for injection to a volume of 10 mL to prepare an injectable agent.

### Formulation Example 23

An agate mortar was used to disperse 2 g of betamethasone into 2 mL of 50% aqueous trehalose, and 0.5 mL of 1% aqueous deacylated gellan gum was further added and mixed. Then it was transferred into a volumetric flask, and 2 mL of 50% aqueous trehalose was further added and agitated using a pencil-type mixer while performing rinse with the same solution, and diluted with water for injection to a volume of 5 mL to prepare an injectable agent.

Formulation compositions of injectable agents from Formulation Examples 20 to 23 are each shown in Table 8.

**[Table 8]**

| | Concentration of betamethasone | Concentration of trehalose | Concentration of gellan gum |
|---|---|---|---|
| Formulation Example 20 | 1% | 0.5% | 0.8% |
| Formulation Example 21 | 30% | 40% | 0.03% |
| Formulation Example 22 | 30% | 25% | 0.1% |
| Formulation Example 23 | 40% | 40% | 0.1% |

### [11] Tests for confirming depot formation

To each glass test tube charged with 15 mL of Dulbecco PBS, 0.020 mL of each injectable agent from Formulation Examples 20 to 23 was added at 37°C. Formation of white depots was visually observed in all test tubes containing the injectable agents from Formulation Examples 20 to 23.

## Claims

1. An injectable agent comprising a medicinal agent, trehalose and gellan gum.

2. The injectable agent according to claim 1, wherein the content of the medicinal agent is 0.0001 to 50.00% (w/v), and the content of trehalose is 0.1 to 50.0% (w/v), and the content of gellan gum is 0.01 to 3.00% (w/v) relative to the total volume of the injectable agent.

3. The injectable agent according to claim 1 or 2, wherein the injectable agent forms a depot upon exposure to a phosphate buffer solution, a body fluid, or a pseudo-body fluid.

4. The injectable agent according to any one of claims 1 to 3, wherein the injectable agent is intended for intravitreal administration.

5. The injectable agent according to any one of claims 1 to 4, wherein the injectable agent is used to deliver the medicinal agent to retina and choroid.

6. A method of forming a depot, the method comprising:
exposing a liquid composition comprising a medicinal agent,
trehalose and gellan gum to a body fluid, a pseudo-body fluid or a phosphate buffer solution.
